Europäisches Patentamt

European Patent Office

Office européen des brevets

(11) Publication number: **0 122 248**
**B1**

# EUROPEAN PATENT SPECIFICATION

(45) Date of publication of patent specification: **18.01.89**

(21) Application number: **84850113.6**

(22) Date of filing: **09.04.84**

(51) Int. Cl.⁴: **G 01 N 1/28, G 01 N 25/14**

(54) A method for analysing gases.

(30) Priority: **12.04.83 SE 8302030**

(43) Date of publication of application:
**17.10.84 Bulletin 84/42**

(45) Publication of the grant of the patent:
**18.01.89 Bulletin 89/03**

(84) Designated Contracting States:
**AT BE CH DE FR GB IT LI LU NL SE**

(56) References cited:
**DE-B-1 925 883**
**GB-A-1 218 493**
**GB-A-1 417 971**

(73) Proprietor: **Boliden Aktiebolag**
**Box 5508**
**S-114 85 Stockholm (SE)**

(72) Inventor: **Rasmusson, Bengt Gunnar Oskar**
**Alstorp 5:64**
**S-240 21 Löddeköpinge (SE)**

(74) Representative: **Lundin, Björn-Eric**
**Boliden Aktiebolag Patents and Trade Marks**
**Box 81550**
**S-104 82 Stockholm (SE)**

Courier Press, Leamington Spa, England.

## Description

Technical Field

The present invention relates to a method for analysing residual gases from large production plants, such as process chemical industry, waste combustion plants, heat and power generators and the like.

The object of the invention is to provide a gas analysing method which enables assays to be made accurately, in a simple and rational fashion, without requiring a large number of gas samples to be taken. A further object of the invention is to provide a method for continuously assaying variations in the contents of a residual gas.

Background Art

It is normal practice to frequently take random samples of a residual gas flow, with the object of controlling the release of harmful substances to atmosphere. This is effected by taking a representative part-flow from the waste-gas outflow, and passing the part-flow through an absorption unit, where the substances sought are removed from the gas. An analysis of the absorbed sample shows the quantity, or quantities, in which the sought substance is, or are present. This quantity is divided by the part-volume of gas withdrawn from the main gas flow, and is adjusted with respect to pressure, temperature and liquid condensate, whereafter the result is multiplied by the total gas flow, so as to obtain the total amount of said substance (substances) in the exiting gas.

When the substance sought in the exiting gas flow is present in the form of liquid droplets, it is necessary to take isokinetic samples of the gas, since an erroneous percentage of droplets results in wide assaying errors. Among other thing, an error in volume can be highly significant when the relationship of droplets to liquid condensate is in error, since 18 g of water in a gas conduit corresponds to a volume of 18 ml, while 18 g of condensate droplets corresponds to a volume of 22000 ml.

The problem created by the presence of liquid droplets is also evident in the case of solid particles, which in a moisture, saturated system are also influenced by the liquid pattern. Significant assaying errors are caused by the fact that particles and gas become enriched in liquid phase.

Isokinetic samples cannot be taken in systems with rotating flows in which the gas is also saturated with moisture. There is no known method for assaying rotating gas flows. The functional control must therefore be based on some other technique, or on a completely novel assaying technique.

In its publication of 1973:8, titled "Riktlinjer för luftvård", Appendix 2, p.31-44, the National (Swedish) Nature Conservancy Board have laid down general guidelines for assaying emissions from air-contaminated plants, and for assessing the result of the assays. Pages 34-38 disclose the manner in which the number of assaying locations is calculated, while page 39 discloses how the sampling nozzles are arranged, and how the assaying equipment is to be organized. Page 39 also discloses that the dust is assayed isokinetically.

When assaying dust manually, the apparatus used comprises a tubular sampling probe, which is inserted in the gas-flow conduit. The probe is coupled to a dust filter, which is followed by a heating apparatus, followed by a gas counter, flow-meter and pump.

It is previously disclosed (DE-B-1,925,883) a method for analyzing gas mixtures, whereby the gas mixture is introduced into a tube shaped heat exchanger and the different components of the gas mixture are allowed to freeze out one by one, whereupon the components are each selectively evaporated for analysis. The method is applied on extremely pure industrial gases, e.g. in the production of semi-conductors.

It is further known (GB-A-1,218,493) to analyze waste gases comprising sulphur trioxide, by introducing a gas mixture comprising sulphur trioxide and other components being insoluble in a condensed steam, into the upper region of a vertical column, which upper region is in contact with a bottom region of a condenser, whereupon superheated steam is introduced into the column, whereby any $SO_3$ present is evaporated off and condensed in said condenser to give a correct value as to the $SO_3$ contents.

Disclosure of the present invention

It has now been surprisingly found possible to achieve good correlation between analysis and actual emission, by mean of the present invention, which is characterized by the steps set forth in the claim.

Thus, emission gases from wet scrubbers treating emission gases from large production process plants, such as chemical industry plants, are withdrawn continuously to recover a stream of sample gas. The sample gas is continuously cooled to about ambient temperature, i.e. about 20°C. A condensate comprising the major part of the water-dissolvable impurity content from the emission gases is thereby obtained. Said impurity content is determined continuously, thereby indicating any variations of the impurity level of the emission gases.

Residual gases from a number of processes contains water in the magnitude of 30-400 g per $m^3$. If a partial flow of gas is cooled to ambient temperature, about 20°C, 25 to 375 ml of a condensate is obtained per $m^3$ of gas thus cooled. In case the gas contains only 30 g of $H_2O$ per $m^3$ the equipment described more in detail below should be provided with a cooler to provide a condensate, whereby such a cooler is not needed at all when the water content is above 40 g per $m^3$. At a continuous cooling the condensate will show a mean value of waste products present, which is proportional against the contents of the residual gas. An increased value in the gas thus gives a corresponding increase in the condensate at constant cooling conditions.

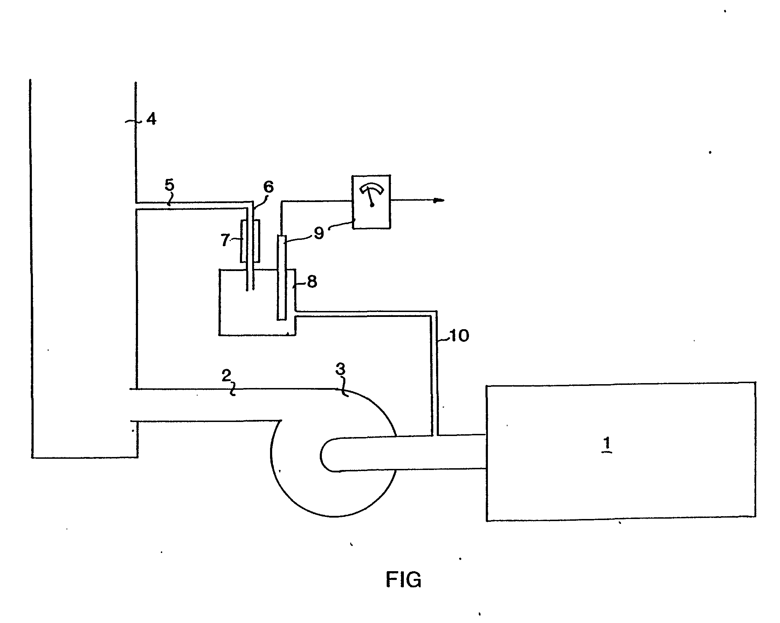

The present invention will now be described in more detail with reference to the accompanying drawing, the single Figure of which illustrates schematically a process industrial plant provided with means for carrying out the present invention.

In the drawing, the reference 1 identifies a gas scrubber, which is connected to a process plant. Residual gases are withdrawn from the gas scrubber through a conduit 2, which is provided with a fan 3 for transporting the residual gas. The conduit 2 discharges into a chimney 4, which discharges the residual gas to atmosphere at a suitable height above ground level, in accordance with current regulations. Arranged at a given distance from the point at which the conduit 2 enters the chimney 4 is a multi-point probe 5, which is connected to a conduit 6. In the event of high ambient temperature, the conduit 6 may have arranged therearound a cooling means 7, in the manner shown. The conduit 6 is connected to a measuring cell 8, for example an electrical conductivity gauge 9, the outlet of which is connected, via a conduit 10, to the gas conduit 2, between the gas scrubber 1 and the fan 3.

During operation, a part-gas flow is drawn through the conduit 6. It will be understood that in its simplest form the conduit 6 may simply extend through the surrounding air and thereby being cooled to ambient temperature. Because the conduit 6 is cool, any water present in the part-gas flow will condense, together with other condensable gases, there being obtained a condensate which runs through the measuring cell 8 containing a conductivity gauge and/or an ion-specific electrode 9. Condensate which has passed through the measuring cell is transported via the gas flow through the line 10, back to the conduit 2, this transportation of the condensate being achieved with the aid of a subpressure in the conduit 2 and the fan 3 arranged therein. The return flow does not disturb the function of the total residual gas outflow.

The condensate obtained flows continuously through the measuring cell 8, thereby enabling the assay to be effected continuously, with storage of the measuring values.

In the case of continuous assaying operations, the measuring cell 8 is a through-flow cell, which may be provided with conductivity-measuring electrodes and/or, in certain cases, ion-specific electrodes. The measuring cell 8 together with its registering means 9 may also be so arranged that when large changes suddenly occur, the amount of through-flowing condensate is transferred to a collecting means, for subsequent particular analysis of the condensate content. Variations due to changes in temperature can also be compensated for.

Furthermore, the sensitivity can be varied with respect to the normal contents measured. Thus, with a variation in sensitivity it is possible to assay over a range of O-10000 mg of emitted product per cubic meter of residual gas.

The registering instrument is normally calibrated to give a normal indication between O-25% of the maximum value. Thus, an increased emission with an increased content of harmful substances will result in a reading which is proportional to the increase of the total emission, or to the increased content of the component for which the indicator is intended.

It has now surprisingly been found that the impurity content of the condensate flow is momentarily proportional to the discharge in the gas scrubber, whereupon elevated contents are obtained with increasing quantities of impurities in the residual gas and also when the supply of water to the gas scrubber is excessively low.

It has also been surprisingly found that by forming a condensate, the impurity content can be concentrated to such an extent as to enable minute quantities of impurities in the residual gas to be detected.

For example, it has been found that the condensate contents obtained were substantially concentrated from 10 to 100 times the expected value. This is particularly pronounced in respect of gases which are soluble in water. This result was achieved in spite of the fact that it is known that the vapour pressure in flowing systems is normally much lower than at equilibrium. It has been established when continuously assaying formed condensate, there is obtained a clear relationship between the contents of the gas and of the condensate chilled out.

With a small liquid volume ($< 10$ ml) an increase of emitted products in the gas phase can be indicated in the condensate within less than 15 min, while a return to a normal content of the gas phase can be established within less than one hour. A still smaller measuring volume has produced a still quicker response, and has made it possible to follow significant variations in gas phase with an extremely short delay.

It has been found that cooling to 20°C is sufficient to satisfactorily monitor and assay the impurity content of emission gases, via a temperature-compensated calculating routine. This means that it is seldom necessary to separately cool the conduit through which part-flow of residual gas is transported. At temperatures of about 20°C the deviation in error is either equal to or much smaller than that afforded when assaying the gas phase isokinetically in the chimney.

When withdrawing a part-flow of the residual gas in accordance with the liquid phase method of the invention, it is unnecessary in a number of cases to measure the amount of gas withdrawn, since it is only desired to assay the content of the condensate.

As before mentioned, when the part-flow of residual gas is accurately cooled, i.e. about 20°C, the major part of the impurities present in the gas and in particle form are transferred to the condensate phase, enabling the total content to be calculated via temperature compensation.

Since the impurity content of the condensate formed corresponds to the impurity content of any droplets present in the gas conduit 2 and the chimney 5, isokinetic sampling has less importance in determining the impurity content of the outgoing gas.

The average impurity content of the condensate phase can be analyzed and multiplied by the amount of water emitted, thereby obtaining the amount of impurities emitted.

When monitoring the emission of the rotating gas flows, a plurality of part-flows should be taken out over a period of such length as to obtain a good mean value for the uneven flow pattern.

In systems where gas and liquid are present under relatively static conditions, the momentary relationship between gas phase and liquid phase can be monitored by means of the present invention. When conditions are favourable, this relationship can be measured directly in the gas flow. In other cases, the relationship is measured in a part-flow withdrawn from the main gas flow.

**Claim**

A method of monitoring the impurity content of emission gases from wet scrubbers treating an outgoing gas from large production plants, said emission gases containing components dissolvable in aqueous systems and at least 30 g of water per m³ comprising continuously sampling the emission gases to recover a stream of sample gas; continuously cooling the sample gas stream to about ambient temperature, thereby forming a condensate comprising the major part of said dissolvable impurity content from the emission gases, and continuously determining the content of impurities present in the condensate to indicate any variations of the impurity level of the emission gases.

**Patentanspruch**

Verfahren zur Überwachung des Verunreini-
gungsgehaltes von Emissionsgasen aus Gasnaß-wäschern, die ein aus großen Produktionsanlagen austretendes Gas behandeln, wobei diese Emissionsgase in waßrigen Systemen lösliche Komponenten und wenigstens 30 g Wasser je Kubikmeter enthalten, indem man kontinuierlich von den Emissionsgasen Proben nimmt, um einen Strom von Probengas zu gewinnen, kontinuierlich den Probengasstrom etwa auf Umgebungstemperatur kühlt, dabei ein Kondensat bildet, das den Hauptteil des löslichen Verunreinigungsgehaltes aus den Emissionsgasen umfaßt, und kontinuierlich den in dem Kondensat vorhandenen Verunreinigungsgehalt bestimmt, um Veränderungen der Verunreinigungskonzentration der Emissionsgase anzuzeigen.

**Revendication**

Procédé de surveillance de la teneur en impuretés des gaz d'émission provenant de laveurs par voie humide traitant un gaz de sortie provenant de grandes installations de production, ces gaz d'émission contenant des composants pouvant être dissous dans des systèmes aqueux et au moins 30 g d'eau par m³, caractérisé en ce qu'il consiste à échantillonner de façon continue les gaz d'émission pour récupérer un courant de gaz d'échantillonnage; à refroidir de façon continue le courant gazeux d'échantillonnage jusqu'aux environs de la température ambiante, pour former ainsi un condensat comprenant la majeure partie de la teneur susdite en impuretés pouvant être dissoutes en provenance des gaz d'émission; et à déterminer de façon continue la teneur en impuretés présentes dans le condensat pour indiquer toutes variations quelconques du niveau d'impuretés des gaz d'émission.

FIG